# EUROPEAN PATENT SPECIFICATION

(11) **EP 1 259 240 B1**
(45) Date of publication and mention of the grant of the patent: **05.07.2006**
(21) Application number: 01900231.0
(22) Date of filing: 09.01.2001
(51) Int. Cl.: A61K 31/455, A61K 31/7004, A61P 17/00

(54) **AGENTS, SUCH AS NICOTINAMIDE OR CADPR FOR THE TREATMENT OF SKIN DISORDERS**
MITTEL, WIE NIKOTINAMIDE ODER CADPR ZUR BEHANDLUNG VON HAUTKRANKHEITEN
Agents de traitement des troubles cutanés, tel le nicotinamide et le cADPR

(30) Priority: 11.01.2000 IL 13397600
(43) Date of publication of application: 27.11.2002
(73) Proprietor: DERMIPSOR LTD, 12 900 Katzrin (IL)
(72) Inventor: DERMIPSOR LTD, 12 900 Katzrin (IL)
(74) Representative: Schmitz, Jean-Marie
(86) International application number: PCT/IL2001/000017
(87) International publication number: WO 2001/051051

(56) References cited:
- EP-A- 0 052 705
- WO-A-98/52529
- DE-A- 4 335 454
- DE-U- 29 916 231
- DATABASE WPI Derwent Publications Ltd., London, GB; AN 1994-170436 XP002183599 FORGACS ET AL: & HU 209 067 B (TATAR J), 28 March 1994 (1994-03-28)
- MORIMOTO ET AL: "an open study of vitamin D3 treatment in psoriasis vulgaris" BRITISH JOURNAL OF DERMATOLOGY, vol. 115, 1986, pages 421-429, XP000905334 cited in the application

## Description

The present invention concerns the use of an agent, the use of a combination.

The present invention is generally concerned with compositions for the treatment of hyperproliferative diseases as well as for the treatment of aging of the epidermis, for use of agents for the preparation of said pharmaceutical compositions as well as for methods of treatment of hyperproliferative diseases and aging of the epidermis.

Normal growth and differentiation of epidermal cells requires a number of regulating factors including Vitamin D3, Vitamin A, a number of cytokines and growth factors, as well as extra- and intracellular free Ca⁺². Skin benign and malignant hyperproliferative disorders arise from faulty regulation of growth and differentiation of epidermal cells. The faulty regulation may be due to lack of response or lack of appropriate response to regulating factors, or due to non-normal levels or function of the regulating factors themselves. For example, it is well known that inappropriate growth and differentiation results from aberrant signaling through the epidermal growth factor receptor. This abnormality may contribute to development of psoriasis, squamous cell carcinomas and of multiple human tumors.

Recently, it was shown that nicotinamide (NA), a derivative of the B vitamin, niacin, can induce differentiation of insulin-producing cells⁽¹⁾. Successful treatment and prevention of insulin dependent diabetes mellitus with NA was demonstrated in animal models⁽²⁾, and therapeutic and prophylactic effects of NA on diabetes mellitus are now in the phase of international clinical trials⁽³⁾.

NA is known as a weak free-radical scavenger, inhibitor of poly-ADP-ribose synthetase and inducible nitric oxide synthase in pancreatic islets⁽⁴⁾. There is single report about the significant role of NA in cellular NAD regeneration after peroxide-induced depletion(5). However the all enumerated effects of NA on human epidermal cells were not studied.

There are many reports of successful treatment of psoriasis and other related skin disorders in humans following oral or topical treatment with vitamin D3 and its analogues⁽⁶⁾.

It is well known that Ca⁺²-signaling Pathways are involved in differentiation of keratinocytes. Recently, cyclic ADP ribose (cADPR), which is a cyclic derivative of NAD+, was discovered as a potent Ca⁺²-mobilizing natural compound in different eukaryotic cells⁽⁷⁾. However, the effect of cADPR on Ca⁺²-signaling in human keratinocytes has not been studied.

Recently it was shown that all-trans-retinoic acid (atRA), which is a vitamin A metabolite induces activation of cADPribose synthesis in renal LLC-PK₁ cells by enhancing activity of ADPR-cyclase without affecting of ADPR-hydrlase⁽⁸⁾. However the effect of atRA on cADPR synthesis in human keratinocytes was not studied.

DE-A-4 335 454 describes a geriatric medical composition in the form of a formulation containing ginseng extracts. Specifically, the composition contains, besides Siberian ginseng extract, Korean ginseng extract and L-carnitine tartrate, also a mixture of vitamins and trace elements.

WO-A-98 52 529 relates to a high level hydration composition, characterized in that it comprises:
a) from 0.01% to 50% preferably from 0.1% to 10%, by weight of a vitamin B3 compound;
b) from 1% to 99% of a conditioning component having a hydration factor of greater than 0, preferably greater than 1.2, more preferably greater than 1.5

EP-A-0 052 705 concerns a composition effective in decreasing the inflammation lesions of acne vulgaris in human patients having such inflammatory lesions, characterized by a carrier containing a therapeutically effect amount of nicotinic acid or nicotinamide.

The present invention is based on the surprising finding that nicotinamide (NA) as well as cyclic, adenosine diphosphate-ribose (cADPR) can promote differentiation, and inhibit proliferation of human epidermal cells in two model systems: a spontaneously immortalized human keratinocyte, termed: "*HaCat cell lines*" which can be used as a model for highly proliferative epidermis, e.g. psoriatic epidermis⁽⁹⁾; and for effects of external modulators of epidermal differentiation⁽¹⁰⁾; and an epidermal carcinoma cell line termed: *"A431* " bearing
the mutated alleles of *p53*, which serves as a model for the testing of anti-cancerogenic drugs⁽¹¹⁾.

The antiproliferative effects of the drugs were demonstrate on rapidly proliferating human keratinocytes, termed *"Cultured Human Epidermal Keratinocytes*", which are used for detecting of antiproliferative treatment of psoriasis⁽¹²⁾.

The present invention is further based on the surprising finding that a composition comprising a mixture of NA and a metabolite of Vitamin D3, being the 1α,25 dihydroxy-vitamin D3 (termed hereinafter: *"1*^{α}*, 25(OH)*_{*2*} *D3*"), is more effective in promoting differentiation and inhibiting proliferation, than the sum of each of the individual effects of NA and the vitamin D3 metabolite when administered separately. This means that the combination of Vitamin D3 metabolite (1α 25(OH₂D3) and NA has a synergistic effect on the differentiation and proliferation of human epidermal cells.

The present invention is further based on the surprising finding that a composition comprising a mixture of all-trans-retinoic acid (atRA), (which is a metabolite of vitamin A), together with NA is more effective in promoting differentiation and inhibiting proliferation of human epidermal cells, than the sum of the effects of each of these compounds separately, i.e. NA and atRA have a synergistic effect in promoting differentiation and inhibiting proliferation of human epidermal cells.

The present invention is further based on surprising findings that long-term NA- treated human keratinocytes are high resistant to hydrogen peroxide-induced oxidative stress, thus indicating that NA may serves as strong antioxidant and therefore a potential anti-aging and anti-cancer protector of human epidermal cells.

Thus, by one aspect the present invention provides a composition for the treatment of hyperproliferative epidermal diseases, and for aging of epidermis comprising a carrier and as an active ingredient or agent selected from the group consisting of:
(i) nicotinamide (NA);
(ii) cyclic adenosine diphosphate-ribose (cADPR); and
(iii) a combination ofNA and cADPR.

The composition may be pharmaceutical or cosmetic.

The composition of the present invention is intended for topical as well as for subcutaneous administrations together with a dermatology or cosmetically acceptable carrier and may be in the form of cream, solution, salve, lotion, ointment or fatty ointment.

The composition may be used to increase the anti-oxidative properties of epidermal cells, for example as an anti-cancer or anti-aging composition.

When used for the treatment of aging epidermis, the cosmetic composition of the invention may be administered as any state of the art cosmetic preparation together with a cosmetically acceptable carrier..

The concentration of nicotinamide in the pharmaceutical composition of the invention should be from 0.5 mM to 20 mM; preferably from 1 mM to 10 mM; most preferably from 2.5 mM to 5 mM.

The concentration of the cADPR in the composition of the invention should be from 1 µM to 100 µM; preferably from 10µM to 50 µM: most preferably from 25 µM to 50 µM.

Where, according to option (iii) above the composition comprises a combination of NA and cADPR the ratio between the two should be from 100:1 to 200:1 (w/w) wherein the final concentration of nicotinamide should be from 2.5 mM to 5 mM and the final concentration of cADPR should be from 25 µM to 50 µM.

By one embodiment of the invention, based on the synergistic activity of NA and a metabolite of Vitamin D3 the composition of the invention comprises a mixture of NA and Vitamin D3 metabolite. Preferably, the vitamin D3 metabolite is 1α,25 dihydroxy-vitamin D₃. The concentration of the NA in the mixture should be within the ranges as defined above. The ratio between the NA and the D3 should be from 5000:1 to 500,000:1 wherein the final concentration of the metabolite vitamin D3 should be from 0.01 µM to 1 µM. Other suitable metabolites of vitamin D3 can be 25-hydroaycholecalciferol (25 OH D3); and 24R. 25-dihydroxycholecalciferol (24R, 25(OH)₂D3).

By a second embodiment, based on the surprising synergistic effect of NA and Vitamin A metabolite (atRA), the present invention concerns a composition comprising NA in the range of concentrations as defined above and a metabolite of vitamin A. preferably by atRA. The final concentration of atRA should be from 0.1 nM to 10 nM. The ratio between NA and atRA should be from 5 x 10⁵:1 to 25 x 10⁷:1.

The term *"hyperproliferative epidermal diseases"* refers to diseases which are characterized by a higher than normal level of proliferation of epidermal cells, and, as a rule, also by abnormal differentiation.

The hyperproliferative epidermal diseases may be malignant: and examples of such malignant diseases are squamous-cell carcinoma (SCC), basal-cell carcinoma (BCC) and other non-melanoma skin cancers (NMSCs).

By another option, the hyperproliferative diseases may be benign diseases such as for example: psoriasis, common warts, keratoacanthoma, seborrhoic keratosis as well as other benign skin disorders like seborrhea or ichthyosis.

The term *"aging of epidermis"* refers to all symptoms associated with physiological aging such as wrinkles, loss of elasticity, decreased metabolism, dryness, roughness, burning and atrophy of skin, itching, heperpilosity and alopecia.

The term "*treatment*" as defined above, refers either to alleviation of the adverse effects of the disease, which alleviation may be manifested, for example, by decrease in the rate of proliferation, improved differentiation, or combination of the two: by complete elimination of the non-normal proliferation and differentiation of the epidermal cells in the diseased person, or alternatively by prevention of the non-normal differentiation and/or higher than normal proliferation before they have occurred. Treatment in the cosmetic aspect may include also prevention of aging signs before they occur. In particular, the compounds of the invention may be used as anti-aging and anti-cancer compositions.

By another aspect the present invention concerns use of an agent selected from the group consisting of:
(i) nicotinamide (NA)
(ii) cyclic adenosine diphosphate-ribose (cADPR); and
(iii) a combination of NA and cADPR
for the preparation of a medicament for the treatment of hyperproliferative epidermal diseases, or for the treatment of aging of the epidermis.

By still another aspect the present invention concerns a method for the treatment of hyperproliferative epidermal diseases or for the treatment of aging of the epidermis comprising: applying to the skin of an individual in need of such treatment, a therapeutically effective amount of an agent selected from the group consisting of:
(i) nicotinamide (NA);
(ii) cyclic adenosine diphosphate-ribose (cADPR); and
(iii) a combination ofNA and cADPR.

The term *"a therapeutically effective amount"* as defined above, refers to an amount which improves, in a measurable manner, either the differentiation of the epidermal cells as determined for example by indirect immunofluorescence analysis of keratin 10 and involucrin expression; by determination of level of envelope cornified formation⁽¹³⁾. Alternatively the amount is an amount which can decrease, to a measurable amount, the proliferation of the cells as indicated by measurement of the activity of mitochondrial dehydrogenase enzymes of living cells (MTT assay)⁽¹⁴⁾ and by counting of basal cells level⁽¹⁵⁾.

The present invention further concerns a method for inhibiting hyperproliferation of epidermal cells comprising contacting the cells with an effective amount of an agent selected from the group consisting of:
(i) nicotinamide (NA);
(ii) cyclic adenosine diphosphate-ribose (cADPR); and
(iii) a combination ofNA and cADPR.

The cells may be malignant epidermal cells such from squamous cell carcinoma (SCC) basal cell carcinoma (BCC) or other non-melanoma skin cancers (NMSC's) or alternatively may be hyperproliferative benign cells, such as human keratiocytes from psoriatic skin, and keratinocytes from keratuacanthoma, common warts or seborrhoic keratoses lesions. The cells may also be from other benign skin disorders such as ichthyosis.

The cells may also be epidermal cells with symptoms of skin ageing (dryness, roughness, burning and atrophy of the skin, itching, cold intolerance, wrinkles, heperpilosity, alopecia), when the epidermal cells are involved in natural, or oxidative stress-inducing aging process. Alternatively, cells may be skin cells having increasing sensitivity to oxidative injury (cells with predisposition to initiation of tumors).

The invention further concerns a method for increasing the anti-oxidative properties of epidermal cells comprising contacting the cells with an effective amount of NA.

In order to understand the invention and to see how it may be carried out in practice, a preferred embodiment will now be described, by way of non-limiting example only, with reference to the accompanying drawings, in which:
**Fig. 1A** shows the effect of NA on HaCat and A431 cell proliferation;
**Fig. 1B** shows anti-proliferative effect of NA on cultured human epidermal keratinocytes;
**Fig. 2** shows the effect of D3 metabolite (la 25(OH)₂D3) on HaCat and A431 cell proliferation;
**Fig. 3** shows the effect of cADPR on HaCat and A431 cell proliferation;
**Fig. 4** shows the effect of Vitamin A metabolite (atRA) on HaCat and A431 cell proliferation:
**Fig. 5A** shows the effect of a combination of NA and D3 metabolite (1α 25(OH)₂D3) on HaCat cell line proliferation;
**Fig. 5B** shows the combined effect of NA and D3 metabolite (1α 25(OH)₂D3) on HaCat cell lines proliferation minus the effect of each of these compounds separately (synergism);
**Fig. 6A** shows the effect of a combination of NA and D3 metabolite (1α 25(OH)₂D3) on A431 cell line proliferation;
**Fig. 6B** shows the combined effect of NA and D3 metabolite (1α 25(OH)₂D3) on A431 cell lines proliferation minus the effect of each of these compounds separately (synergism);
**Fig. 7A** shows the effect of a combination of NA and cADPR on HaCat cell line proliferation;
**Fig. 7B** shows the combined effect of NA and cADPR on HaCat cell lines proliferation minus the effect of each of these compounds separately (synergism);
**Fig. 8A** shows the effect of a combination of NA and cADPR on A431 cell line proliferation;
**Fig. 8B** shows the combined effect of NA and cADPR on A431 cell lines proliferation minus the effect of each of these compounds separately (synergism);
**Fig. 9A** shows the effect of a combination of NA and Vitamin A metabolite (atRA) on HaCat cell line proliferation;
**Fig. 9B** shows the combined effect of NA and Vitamin A metabolite (atRA) on HaCat cell lines proliferation minus the effect of each of these compounds separately (synergism);
**Fig. 10A** shows the effect of a combination of NA and Vitamin A metabolite (atRA) on A431 cell line proliferation;
**Fig. 10B** shows the combined effect of NA and Vitamin A metabolite (atRA) on A431 cell lines proliferation minus the effect of each of these compounds separately (synergism);
**Fig. 11** shows the effect of NA on involucrin and keratin k10 expression in HaCat cells;
**Fig. 12** shows the effect of NA on basal and envelope cornified cell expression in HaCat cell line;
**Fig. 13** shows the effect of NA on apoptosis level in HaCat and A431 cell lines; and
**Fig. 14** shows the resistance of HaCat cells treated for long-term period with NA to oxidative stress induced by hydrogen perioxide (H₂O₂).

### DETAILED DESCRIPTION OF THE INVENTION

### EXPERIMENTAL PROCEDURES

### A. Cell culture

The immortalized human keratinocyte HaCat cells were routinely cultured in 75 cm² flasks using Eagle's minimal essential medium (MED-EAGLE) supplemented with 5% fetal calf serum (FCS) and 1% of antibiotics (penicillin 20 u/ml; streptomycin 20 µg/ml and nystatin 2.5 u/ml) at 37°C in 95% air/5% CO₂. Medium was changed every 3-4 days. In some experiments HaCat cells cultivated for 6 months in routinely used medium supplemented with 10 mM NA or 20 mM NA (long-term culture of HaCat cells with NA) were used.

Human Epidermal Keratinocytes (passages 3-6) obtained from normal face-lift surgery were cultivated in serum-free KGM® -2 BulletKit® CC-3107 (Clonetics, USA) medium with low calcium for accelerated proliferation of the keratinocytes. Epidermal carcinoma A431 cell line was cultured in DMEM medium supplemented with 10% FCS and antibiotics.

### B. Reagents

Nicotinamide (NA); cyclic adenosine diphosphate-ribose (cADPR); calcitriol (1α, 25-dihyroxy-vitamin D3); all trans retinoic acid (atRA; Vitamin A acid: Tretinoin): 3-(4,5-dimethylthiazol-2-yl)-2,5-diphenyltetrazolium bromide (MTT): propidium iodide; dimethylsulphoxide (DMSO); bovine serum albumin (BSA): sucrose: trisodium citrate; igepal CA-630 (NP-40); Tris- (hydroxymethyl)-aminomethane; trypsin; trypsin inhibitor; ribonuclease A; spermintetrahydrochloride: sodium dodecylsulfate (SDS); β-mercaptoethanol and hydrogen peroxide (H₂O₂) were obtained from Sigma (USA). Eagle's minimal essential medium (MEM-EAGLE); 2 MEM antibiotics; fetal calf serum (FCS); L-glutamine; Dulbecco's phosphate buffered saline (PBS); trypsin 0.05%-EDTA solution (1:000) were obtained from Biological Industries (Israel). Keratinocyte Growth Medium® -2 Bullet Kit® CC-3107 (for accelerated proliferation) was received from BioWhittaker, Inc. A Cambrex Company, Clonetics, USA). Anti-human cytokeratin 10 (NCL-CK10) and involucrin (NCL-INV) mouse monoclonal antibodies were obtained from Novocastra Laboratories Ltd. (UK) and Cy^{™} 2-conjugated goat anti-mouse IgG from Jackson Immunoresearch Laboratories, Inc. (USA). HaCat and A431 cells were propagated in 25 cm² or 75 cm² tissue culture flasks (Coming, USA) and 24-well and 96-well tissue culture plates (Coming, USA) were used for incubation of the cells with different doses of NA (1-50 mM/I), cADPR (1-50 microM), Vitamin D3 (1-10000 nM) and atRA (0.1-10000 nM).

### C. Proliferation assays

### C1 MTT method

Viability/proliferation of HaCat and A431 cells and Cultured Human Epidermal Keratinocytes after treatment with various concentrations of nicotinamide alone and in combination with cADPR, Vitamins D3 and A metabolites was determined by MTT assay (Mosmann, T: Rapid colorimetric assay for cellular growth and survival: Application to proliferation and cytotoxicity assays, *J. Immunol Meth*.,**65**:55-63, (1983)) in 96-well microtiter plates. Briefly, an equal number of the cells were seeded in each well and after 24 h of incubation NA alone or in different combinations (NA and cADPR; NA with D3; NA with Vitamin A) was added at the various final concentrations. After 72 h of incubation 20 µl of 5 mg/ml MTT in phosphate buffered saline (PBS) without Ca⁺² and Mg⁺² were added to each well. Plates were then placed in an incubator CO₂ and MTT was converted t the insoluble MTT-formazan crystals by mitochondrial dehydrogenases during 3.5 h. Medium was removed and formazan crystals were dissolved in 0.2 ml of DMSO. The amount of formazan was quantified in an ELISA-reader at 550 nm. Background values at 650 nm were subtracted. Data are results from three independent experiments.

### D. Differentiation assays

### D1 Cornified Envelope Formation

Cornified cell envelope formation was determined (Sun T-T, Green, H: Differentiation of the epidermal keratinocytes in cell culture: formation of cornified envelope, *Cell*, **9**:511-521, 1976 as a measure of late differentiation processes in HaCat cells treated with nicotinamide. Briefly, cells were seeded in 24-well tissue culture plates and after attachment (24 h) exposed to various concentrations of NA (0, 4, 10. 15 and 20 mM) for 96 h. The cells were detached and resuspended in medium. Counting of total and basal (small, rounded) cells was performed using hemocytometer in tetraplicate aliquotes. The remaining cells were spun down, treated with 10 mM Tris-HCl (pH 7.4) supplemented with 1% β-mercaptoethanol and 1% SDS for 10 min and cornified envelope cells were counted in tetraplicate aliquots using hemocytometer. Results from three independent experiments were presented.

### D2 Indirect immunofluorescence

Effects of NA on early (keratin k10 expression) and late (involucrin expression) differentiation processes on HaCat cells were estimated by indirect immunofluorescence. Briefly 2x10⁴ cells/ml were seeded on glass coverslips into Petri dishes with 0, 5, 10 and 20 mM NA. After 72 h of incubation cells on the glass coverslips were washing with PBS, fixed by ice-cold methanol: acetone (1:1) and incubated at -20°C for 10 minutes. Fixed cells were washed in PBS and incubated with blocking buffer (1% BSA in PBS) for 10 minutes to minimize nonspecific absorption of the primary antibodies to the coverslips. To detect the keratin 10 expression antihuman mouse monoclonal antibody was used at 1/50 final dilution. Involucrin expression was detected by antihuman involucrin mouse monoclonal antibody in final dilution 1/100. After blocking the cells were incubated with the aforementioned antibodies at 37°C for 1 h in a humidified chamber. Exhaustive washed with PBS cells were incubated with Cy^{™} 2-conjugated goat anti-mouse IgG in final dilution 1/50 for 30 min. at room temperature. Slides were viewed under Zeiss microscope (Axioskop-2) equipped with epifluorescence optics and the appropriate filters to avoid cross-channel contamination. Level of keratin 10 and involucrin expression was estimated by counting the positive cells relative to the total cell number. In each slide at least 500-1000 cells were scored. The data presents mean of 3 independent experiments.

### D3 DNA labeling and flow cytometry analysis

HaCat and A431 cells were seeded in 25 cm² tissue culture flasks and incubated for 72 h with 0, 5, 10 and 20 mM of NA. Cells treated with 5% ethanol served as positive control of apoptosis. The nuclei for flow cytometry analysis of DNA were prepared by a detergent trypsin method with propidium iodide (Lars L Rindelov: A detergent trypsin method for the preparation of nuclei for FACS DNA anlaysis, *Cytometry* **3**(5)323-327, 1983). Briefly, the cells (10⁶ per tube) were washed with PBS. The cell pellet was resuspended in 40 µl citrate buffer (pH 7.6) supplemented with 250 mM sucrose, 40 mM trisodium citrate and 5% DMSO. Then resuspended cells were incubated in 450 µl solution with trypsin (0.15 mg/ml, pH 7.6) for 10 minutes. Following next 10 min. incubation with trypsin inhibitor and ribonuclease A the fluorochrome solution containing propidium iodide 100 µg/ml was added to nuclei. The tubes were placed in the dark before flow cytometry analysis. Flow cytometry analysis was carried out in fluorescence-activated cell sorter (FACScan; Becton Dickinson, CA) and level of apoptosis was determined using the Cell Quest Program of Becton Dickonson. Each experiment was repeated three times.

### E. Statistical analysis

Results are presented as mean ± standard deviation of the mean (mean±SD). Statistical significance (P<0.05) was derived by Student's t-test.

### Example 1 NA effect on epidermal cell proliferation

2x10⁴/ml immortalized human keratinocyte HaCat cells, Cultured Human Epidermal Keratinocytes and 5x10³/ml squamous carcinoma A431 cells were incubated with varying amounts of NA for a period of 72 hours. The proliferation was estimated by the MTT method, as described in Section C of Experimental Procedures, and was expressed as the percent from control (untreated cells).

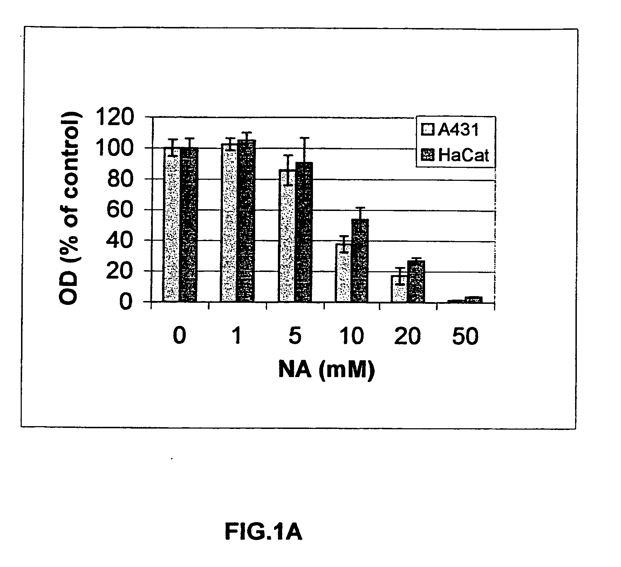

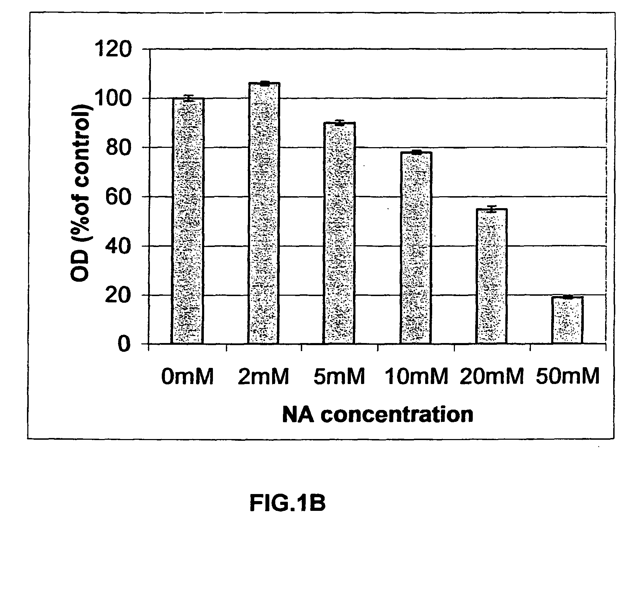

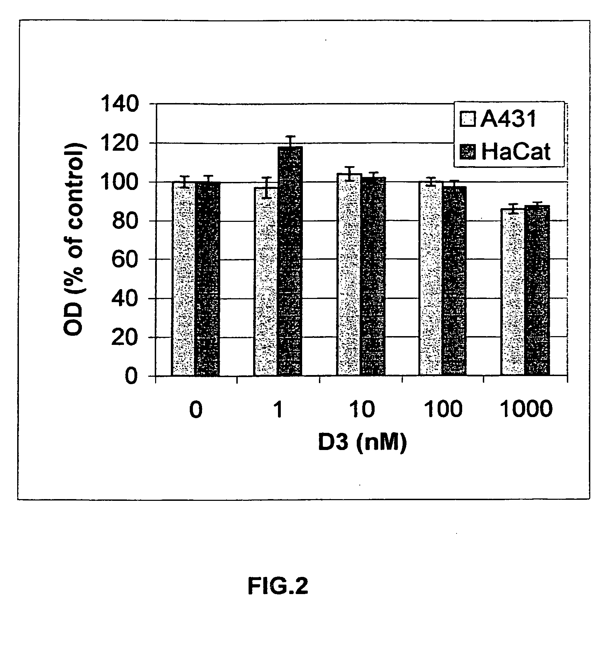

The results are shown in Fig. 1A and 1B, which indicates that both HaCat, A431 and Cultured Human Epidermal Keratinocyte cell proliferation was significantly inhibited by NA.

### Example II Effect of cADPR Vitamin D3 metabolite, and Vitamin A metabolite on cell proliferation

Cells as described in Example 1, were incubated with varying amounts of cADPR for 72 hours.

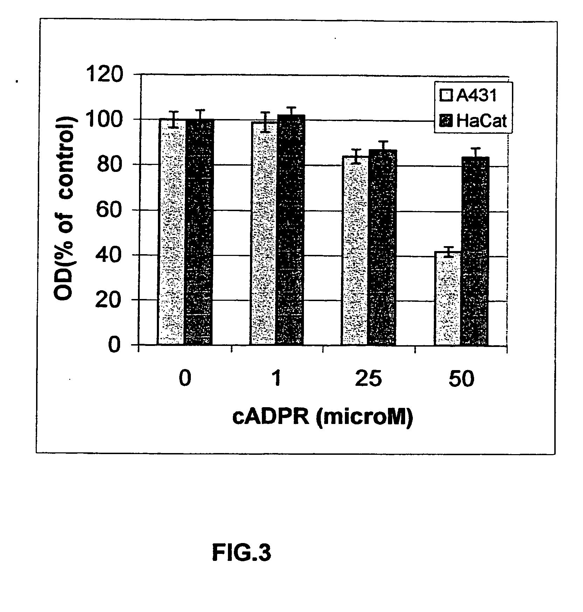

As can be seen in Fig. 3, 25 and 50 µM of cADPR were effective in inhibiting proliferation.

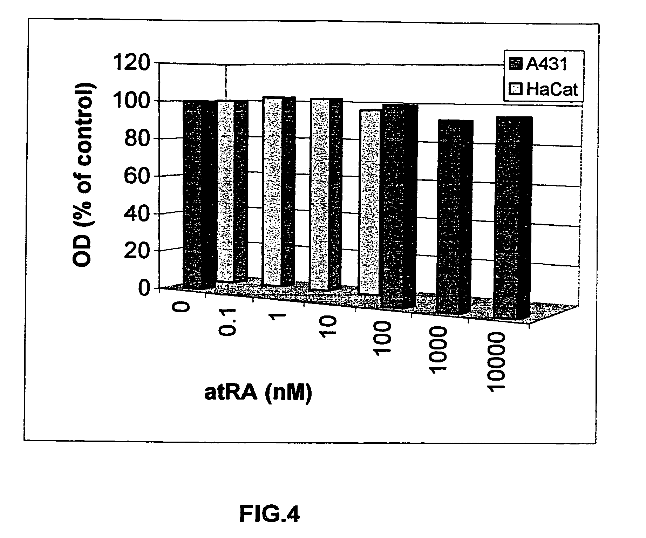

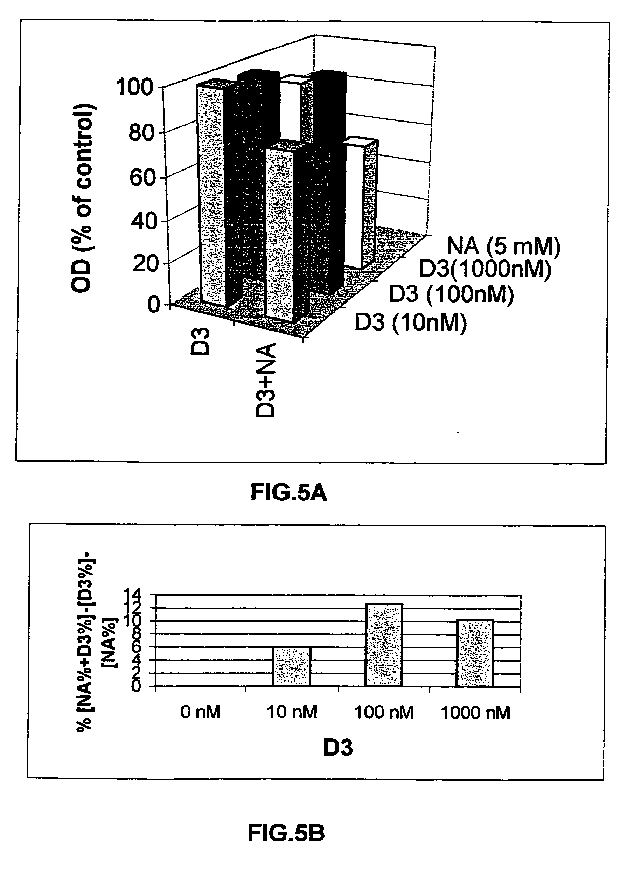

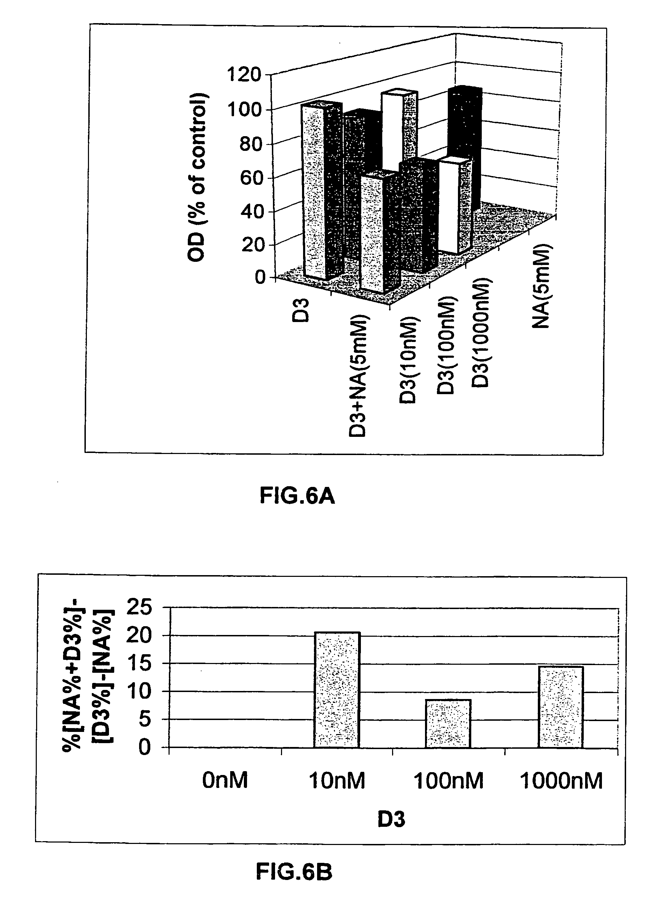

Effects of Vitamin D3 metabolite (1α 25(OH)₂D3), and Vitamin A metabolite (atRA) on cell proliferation were determined. Cells, as described above were incubated with varying amounts of Vitamin D3 metabolite and Vitamin A metabolite, and the results are shown in Figs. 2 and 4, respectively.

As can be seen, in the concentrations examined, neither Vitamin D3 metabolite nor Vitamin A metabolite, alone, affected the proliferation of HaCat and A431 cell line.

### Example 3 Synergistic effects of NA and a Vitamin D3 metabolite

HaCat cells and A431 cells were incubated with the D3 metabolite separately, and the D3 metabolite together with NA (Figs. 5A and 6A, respectively) for a period of 72 hours.

The effect of each of D3 metabolite and NA separately was deduced from the combined effect ofNA together with the D3 metabolite in HaCat cells (Fig. 5B) and A431 cells (Fig. 6B).

As can be seen, the combined effect of NA and D3 metabolite was higher than the summation of each of these effects separately, in an amount of above 12% for a concentration of 100 nM of D3 and 5 mM NA in respect of HaCat cells, and above 20% synergism for 10 nM of D3 metabolite and 5 mM NA for A431 cells.

### Example 4 Synergistic effect of cADPR and NA

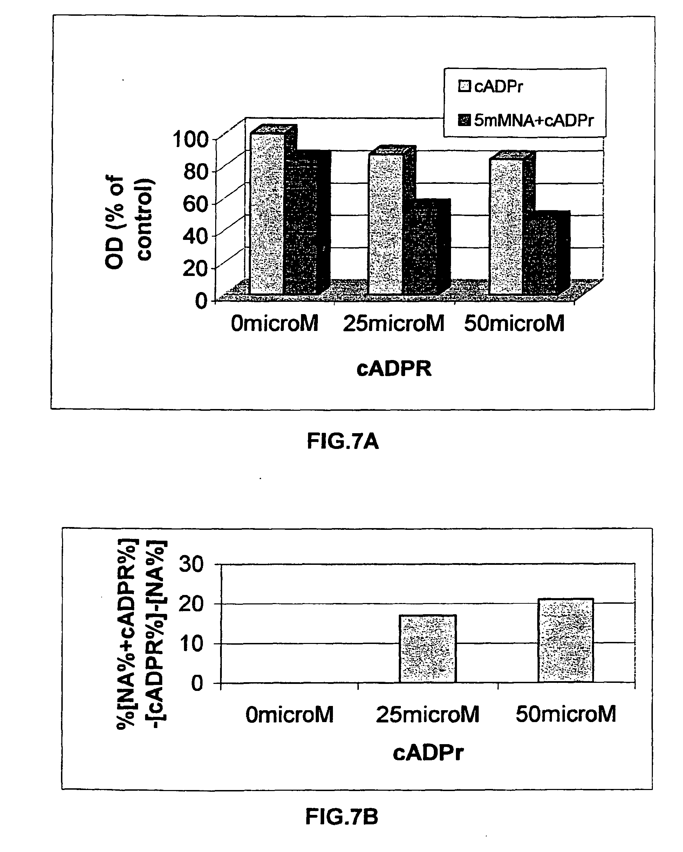

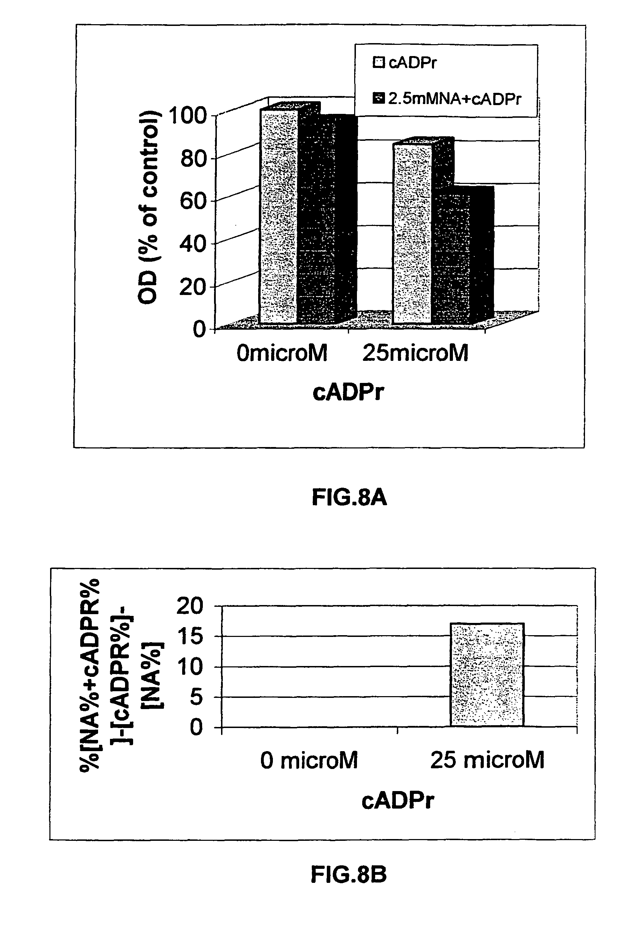

2x10⁴/ml HaCat cells and 5x10³/ml squamous carcinoma A431 cells were incubated with NA at a concentration of 5 mM and 2.5 mM respectively, varying amount of cADPR (25-50 µm) and the results are shown in Figs. 7 and 8 respectively.

As can be seen from Fig. 7A, 50 µM of cADPR and 5 mM ofNA showed a synergistic effect of above 20% in inhibiting proliferation as compared to the effect of each of these agents in that concentration alone in respect of HaCat cells, and 25 µM of cADPR and 2.5 mM NA showed a synergism of above 16% in inhibiting proliferation of A431 cells, as compared to the inhibition of each of these components alone.

### Example 5 Synergistic effect of a combination of NA and Vitamin A metabolite on epidermal cells

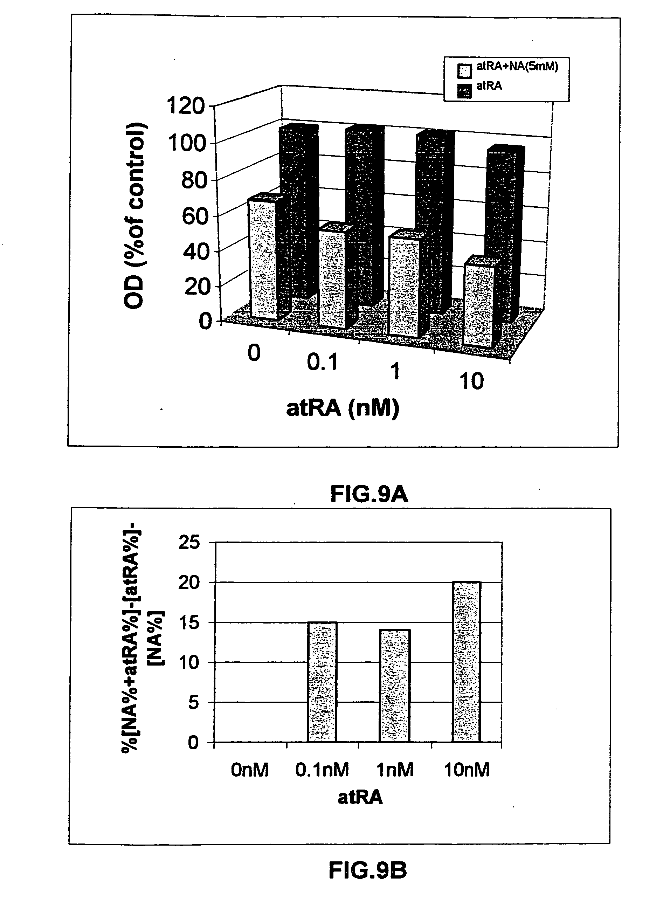

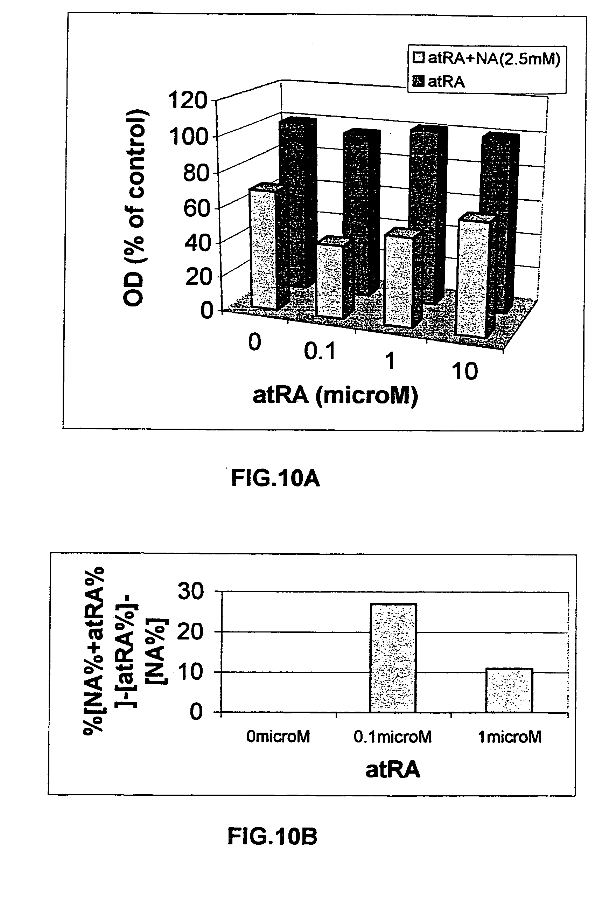

HaCat cells and A431 cells were incubated with NA at an amount of 5 mM and 2.5 mM respectively and varying concentrations of atRA (0.1 nM-1 µM) and the results are shown in Figs. 9 and 10, respectively.

As can be seen in Figs. 9B and 10B, the effect of the combination showed a synergism of above 25% at a concentration of 0.1 µM atRA for A431 cell (Fig. 10B) and a synergism of above 20% for a concentration of 10 µM of atRA (for HaCat cells) (Fig. 9B), above the effect achieved for each of the separate agents Vitamin A and NA in the same amounts separately.

### Example 6 Effect of NA on cell differentiation and apoptosis

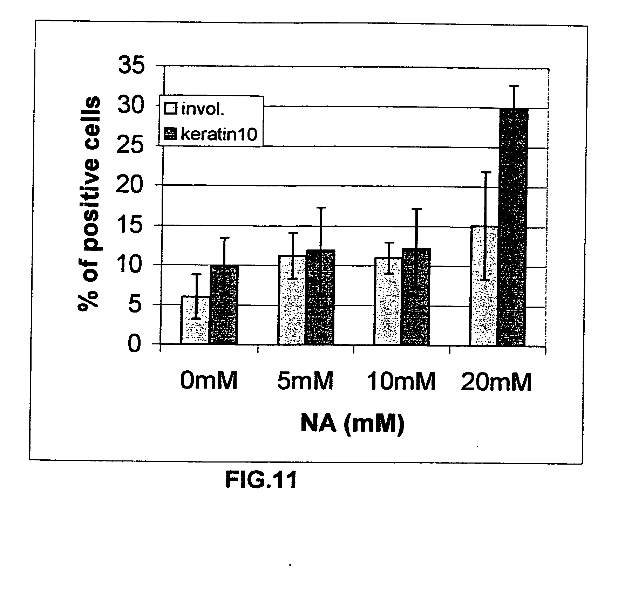

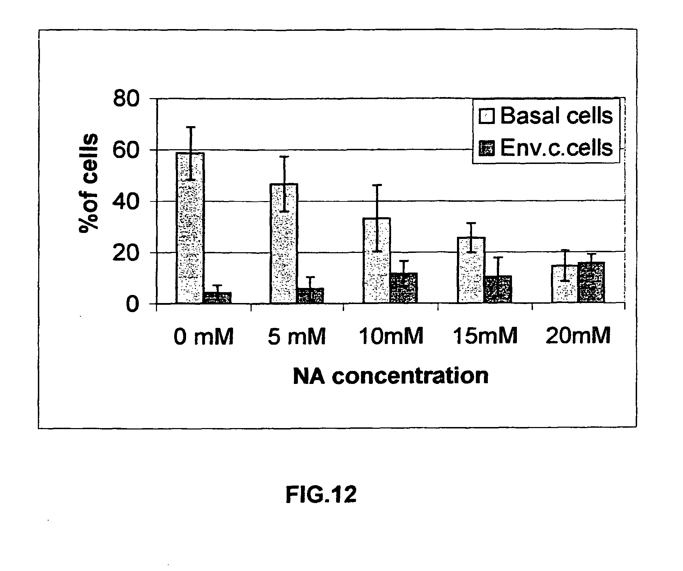

The effect of NA on differentiation was determined by cornified envelope formation as described in D 1 and indirect immunofluorescence of keratin K10 and involucrin as described in D2 and the results are shown in Figs. 11 and 12, respectively.

As can be seen, NA treatment, simulated both expression of keratin 10 (K10) involucrin, which are markers of early and late differentiation processes of the epidermal cells.

NA, also changed the ratio between the amount of cells, and envelope cornified cells, so that a higher proportion of cells tested were enveloped cornified cells, which are more differentiated cells, as can be seen in Fig. 12.

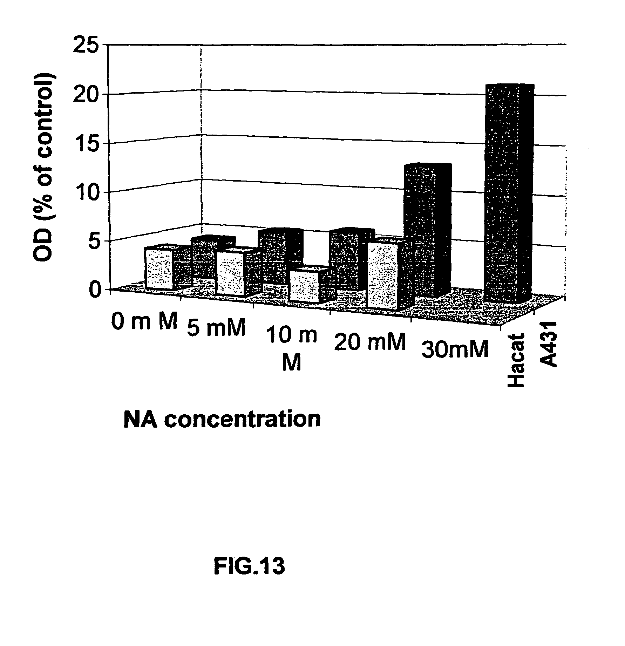

Finally, Fig. 13 shows that NA becomes cytotoxic to cell as determined by the level of apoptosis in amounts of 30 mM for A431 cells and 50 mM for HaCat cells. This means that the effect of NA on cell proliferation, as for example, expressed in Fig. 1 is manifested in concentration of NA which are below the concentrations toxic to cells.

### Example 7 Resistance of HaCat cells long-term cultured with NA (10 mM) to hydrogen peroxide-induced oxidative stress

2x10⁴/ml immortalized human keratinocyte HaCat cells cultivated routinely, or the same amount of HaCat cells cultured with 10 mM NA during 6 months, were incubated with increasing concentrations of hydrogen peroxide for period of 24 hours. The cytotoxicity was estimated by the MTT method, as described in Section C of "Experimental Procedures", and was expressed as the percent from control (untreated cells).

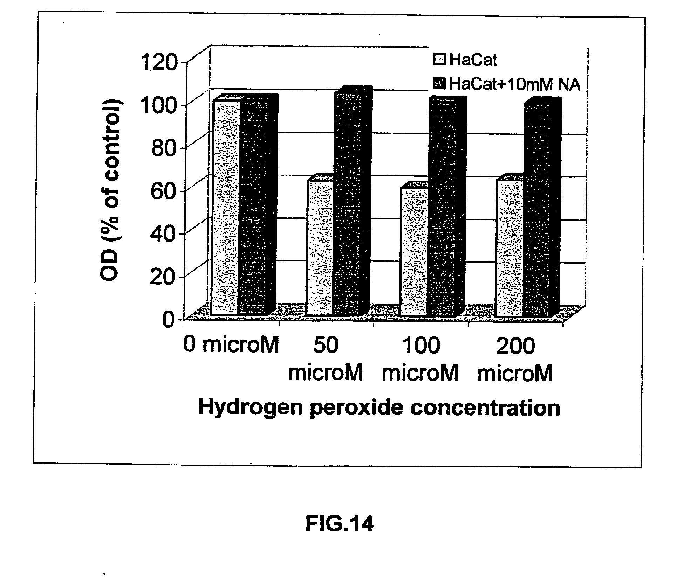

The results are shown in Fig. 14, which demonstrates that only HaCat cells cultivated routinely (without NA supplementation) were significantly injured by hydrogen peroxide. These data indicate that long-term treatment with NA may increase anti-oxidative properties of human epidermal cells.

### REFERENCES

1. Otonkoski T., Beatie, G.M., Mally, M.I., Ricordi, S., Hayek, A., *"Nicotinamide is a potent inducer of endocrine differentiation in cultured human fetal pancreatic cells", J. Clin. Invest.,* **92**:1459-1466, (1993).
2. Yamada, K., Nonaka, K., Hanafusa, T., Miyazaki, A., Toyoshima, H., Tarui, S: *"Preventive and therapeutic effects of large-dose nicotinamide injections on diabetes associated with insulitis: an observation in nonobese diabetic (NOD) mice", Diabetes,* **31**:749-753, (1982).
3. Elliot, R.B., Chase, H.P.: *"Prevention or delay of Type I (insulin-independent) diabetes mellitus in children using nicotinamide". Diabetologia,* **34**:362-365, (1991).
4. Petley, A., Macklin, B., Renwick, A.G., Wilkin, T. J.: *"The Pharmacokinetics of Nicotinamide in Human and Rodents". Diabetes,* **44**:152-155,(1995).
5. Grant, R.S., Kapoor, V.: *"Murine glial cells regenerate NAD, after peroxide-induced depletion, using either nicotinic acid, nicotinamide, or quinolinic acid as substrates ", J. Neurochem.,* **70**:1759-1763, (1998).
6. Morimoto S., Yoshikawa, K., Konzuka, T., *et al.. "An open study of vitamin D3 treatment in psoriasis vulgaris", Br. J. Dermatol.,* **115**:421-429, (1986).
7. Lee HC, Specific binding of cyclic ADP-ribose to calcium-storing microsomes from sea urchin eggs. *J. Biol. Chem*., **266**:2276-2281 (1991).
8. Beers K.W., Chini, E.N., and Dousa, T.P.: *"All-trans-retinoic acid stimulates synthesis of cyclic ADP-ribose in renal LLC-PK*_{*1*}*", J. Clin. Invest*., **95**:2385-2390, (1995).
9. Ockenfels. H.M., Nuβbaum, G., Schultewolter, T., Burger, PM., Goos, M.: *"Cyclosporin A, FK506 and dithranol alter tyrosine-specific protein phosphorylation in HaCat keratinocytes". Arch. Dermatol. Res.,* **287**:304-309, (1995).
10. Paramio. J. M., and Jorcano, J.L.: *"Role of protein kinases in the in vitro differentiation of human HaCat cells". Brit. J. Dermatol*., **137**:44-50, (1997)).
11. Ahmad, N.. Feyes, D.K., Agarwal, R., Mukhtar, H: Photodynamic therapy results in induction of WAF1/CIPI/P21 leading to cell cycle arrest and apoptosis. *Proc. Natl. Acad. Sci. USA,* **95**:6977-6982, (1998).
12. Nikoloff, B.J., Fisher, G.J., Mitra, R.S., Voorhees, J.J.: *"Additive and Synergistic Antiproliferative Effect of Cyclosporin A and Gamma Interferon on Cultured Human Keratinocytes ". Amer. J. Pharmacol.,* **131**:12-18, (1988).
13. Sun T-T, Green, H: Differentiation of the epidermal keratinocyte in cell culture: formation ofcomified envelope, *Cell*, **9**:511-521, (1976).
14. Mosmann, T: Rapid colorimetric assay for cellular growth and survival: Application to proliferation and cytotoxicity assays. *J. Immunol. Meth*., 65:55-63, (1983).
15. Sun T-T, Green, H: Differentiation of the epidermal keratinocyte in cell culture: formation of cornified envelope (*Cell,* **9**:511-521 (1976).

## Claims

1. Use of an agent selected from the group consisting of
(i) nicotinamide (NA);
(ii) cyclic adenosine diphosphate-ribose (cADPR); and
(iii) a combination of NA and cADPR
for the preparation of a composition for the treatment of psoriasis, ichythyosis or malignant hyperproliferative epidermal diseases.

2. Use of a combination of NA and at least one Vitamin D3 metabolite for the preparation of a medicament for the treatment of psoriasis, ichythyosis or malignant hyperproliferative epidermal diseases.

3. Use according to Claim 2, wherein the metabolite of the Vitamin D3 is *1α,25* dihydroxy vitamin D3.

4. Use of a combination of NA and Vitamin A metabolite for the preparation of a medicament for the treatment of psoriasis, ichythyosis or malignant hyperproliferative epidermal diseases.

5. Use according to Claim 4, wherein the Vitamin A metabolite is all-trans-retinoic acid (atRA).

6. Use according to any one of claims 1 to 5, wherein the malignant disease is selected from the group consisting of squamous-cell carcinoma (SCC), basal-cell carcinoma (BOC) and other non-melanoma skin cancers (NMSCs).

## Patentansprüche

1. Verwendung eines Mittels, ausgewählt aus der Gruppe, bestehend aus
(i) Nikotinamid (NA);
(ii) zyklischer Adenosindiphosphat-Ribose (cADPR); und
(iii) einer Kombination aus NA und cADPR
für die Herstellung einer Mischung zur Behandlung von Psoriasis, Ichthyosis oder malignen hyperproliferativen Epidermiskrankheiten.

2. Verwendung einer Kombination aus NA und wenigstens einem Vitamin-D3-Metabolit für die Herstellung eines Arzneimittels zur Behandlung von Psoriasis, Ichthyosis oder malignen hyperproliferativen Epidermiskrankheiten.

3. Verwendung nach Anspruch 2, wobei der Metabolit des Vitamins D3 *1α,25* Dihydroxyvitamin D3 ist.

4. Verwendung einer Kombination aus NA und Vitamin-A-Metabolit zur Herstellung eines Arzneimittels zur Behandlung von Psoriasis, Ichthyosis oder malignen hyperproliferativen Epidermiskrankheiten.

5. Verwendung nach Anspruch 4, wobei der Vitamin-A-Metabolit All-Trans-Retinol-Säure (atRA) ist.

6. Verwendung nach einem der Ansprüche 1 bis 5, wobei die maligne Krankheit ausgewählt ist aus der Gruppe, bestehend aus Plattenepithelkarzinom (SCC), Basalzellenkarzinom (BCC) und sonstigen nicht-melanomen Hautkarzinoma (NMSC).

## Revendications

1. Utilisation d'un agent choisi parmi le groupe constitué par :
(i) le nicotinamide (NA) ;
(ii) l'adénosine diphosphate ribose cyclique (cADPR) ; et
(iii) une combinaison de NA et de cADPR
pour la préparation d'une composition destinée au traitement du psoriasis, de l'ichtyose ou encore de maladies épidermiques hyperprolifératives malignes.

2. Utilisation d'une combinaison de NA et d'au moins un métabolite de la vitamine D3 pour la préparation d'un médicament destiné au traitement du psoriasis, de l'ichtyose ou de maladies épidermiques hyperprolifératives malignes.

3. Utilisation selon la revendication 2, dans laquelle le métabolite de la vitamine D3 est la *1α, 25* dihydroxy vitamine D3.

4. Utilisation d'une combinaison de NA et d'un métabolite de la vitamine A pour la préparation d'un médicament destiné au traitement du psoriasis, de l'ichtyose ou de maladies épidermiques hyperprolifératives malignes.

5. Utilisation selon la revendication 4, dans laquelle le métabolite de la vitamine A est l'acide rétinoïque tout trans (atRA).

6. Utilisation selon l'une quelconque des revendications 1 à 5, dans laquelle la maladie maligne est choisie parmi le groupe constitué par l'épithélioma malphigien spinocellulaire (SCC), l'épithélioma basocellulaire (BCC) et d'autres cancers de la peau sans présence d'un mélanome (NMSC).
